# EUROPEAN PATENT SPECIFICATION

(11) **EP 3 931 571 B1**
(45) Date of publication and mention of the grant of the patent: **07.06.2023**
(21) Application number: 20706332.2
(22) Date of filing: 28.02.2020
(51) Int. Cl.: G01N 33/574

(54) **PROTEIN SIGNATURE FOR THE DIAGNOSIS OF COLORECTAL CANCER AND/OR PRE-CANCEROUS STAGE THEREOF**
IN-VITRO-VERFAHREN ZUR DIAGNOSE VON KOLOREKTALEM KREBS UND/ODER DESSEN PRÄKANZEROGENEM STADIUM
PROCÉDÉ IN VITRO POUR LE DIAGNOSTIC DU CANCER COLORECTAL ET/OU DE SON STADE PRÉCANCÉREUX

(30) Priority: 01.03.2019 EP 19382156; 01.03.2019 EP 19382157
(43) Date of publication of application: 05.01.2022
(73) Proprietor: Advanced Marker Discovery, S.L., 47004 Valladolid (ES)
(72) Inventor: MARTÍN RODRÍGUEZ, Ana Carmen, 47004 Valladolid (ES); PÉREZ PALACIOS, Rosa, 47004 Valladolid (ES); ARROYO ARRANZ, Rocío, 47004 Valladolid (ES)
(74) Representative: Hoffmann Eitle
(86) International application number: PCT/EP2020/055277
(87) International publication number: WO 2020/178172

(56) References cited:
- US-A1- 2014 220 006
- HONGDA CHEN ET AL: "Development and validation of a panel of five proteins as blood biomarkers for early detection of colorectal cancer", CLINICAL EPIDEMIOLOGY, vol. Volume 9, 1 October 2017 (2017-10-01) , pages 517-526, XP055512097, DOI: 10.2147/CLEP.S144171
- H. CHEN ET AL: "Head-to-Head Comparison and Evaluation of 92 Plasma Protein Biomarkers for Early Detection of Colorectal Cancer in a True Screening Setting", CLINICAL CANCER RESEARCH, vol. 21, no. 14, 26 May 2015 (2015-05-26), pages 3318-3326, XP055512107, US ISSN: 1078-0432, DOI: 10.1158/1078-0432.CCR-14-3051
- CHEN HONGDA ET AL: "Empirical evaluation demonstrated importance of validating biomarkers for early detection of cancer in screening settings to limit the number of false-positive findings", JOURNAL OF CLINICAL EPIDEMIOLOGY, PERGAMON, GB, vol. 75, 2 February 2016 (2016-02-02), pages 108-114, XP029616862, ISSN: 0895-4356, DOI: 10.1016/J.JCLINEPI.2016.01.022
- QIAN JING ET AL: "Biomarker discovery study of inflammatory proteins for colorectal cancer early detection demonstrated importance of screening setting validation", JOURNAL OF CLINICAL EPIDEMIOLOGY, PERGAMON, GB, vol. 104, 1 August 2018 (2018-08-01), pages 24-34, XP085523437, ISSN: 0895-4356, DOI: 10.1016/J.JCLINEPI.2018.07.016

## Description

### FIELD OF THE INVENTION

The present invention relates to diagnosis of colorectal cancer or a pre-cancerous stage thereof using the protein signature [Flt3L and CYFRA21-1] in blood, serum or plasma.

### STATE OF THE ART

Colorectal cancer (CRC) (also known as colon cancer, rectal cancer, or bowel cancer) is the development of cancer in the colon or rectum (parts of the large intestine). The vast majority of colorectal cancers are adenocarcinomas. This is because the colon has numerous glands within the tissue. When these glands undergo a number of changes at the genetic level, they proceed in a predictable manner as they move from benign to an invasive, malignant colon cancer. The adenomas of the colon, particularly advanced colorectal adenoma (AA), are a benign version of the malignant adenocarcinomas but still with malignant potential if not removed (they are usually removed because of their tendency to become malignant and to lead to colon cancer).

Screening is an effective way for preventing and decreasing deaths from colorectal cancer and is recommended starting from the age of 50 to 75. The best known and most frequently used screening test for colorectal cancer is called Fecal Immunochemical Test (FIT). FIT detects blood in the stool samples which can be a sign of pre-cancer or cancer. If abnormal results are obtained, usually a colonoscopy is recommended which allows the physician to look at the inside of the colon and rectum to make a diagnosis. During colonoscopy, small polyps may be removed if found. If a large polyp or tumor is found, a biopsy may be performed to check if it is cancerous. The gastroenterologist uses a colonoscopy to find and remove these adenomas and polyps to prevent them from continuing to acquire genetic changes that will lead to an invasive adenocarcinoma.

Although, as explained above, FIT is nowadays used for screening colorectal cancer, it is important to note that FIT offers a low sensitivity for AA (around 20-30% depending on literature) which means that most of said kind of patients can be wrongly classified as not having the disease. Consequently, FIT is not able to identify adenomas due to its low sensitivity. Moreover, since FIT uses stool samples, it offers a low compliance. On the other hand, the colonoscopy is an invasive technique wherein the most severe complication generally is the gastrointestinal perforation. On the other hand, colonoscopy is nowadays a procedure involving anesthesia, and the laxatives which are usually administered during the bowel preparation for colonoscopy are associated with several digestive problems.

It is important to note that the methods used today for screening general population at risk of suffering for CRC or AA are associated with a high rate of false positives. Consequently, a high amount of unnecessary follow-up colonoscopies are nowadays performed.

Chen et al. (Clin Epidemiol. 2017; 9: 517-526) discloses a multi-marker panel of 5 blood-based protein markers for diagnosing colorectal cancer: growth differentiation factor 15 (GDF-15), amphiregulin (AREG), Fas antigen ligand (FasL), Fms-related tyrosine kinase 3 ligand (FLT3L) and TP53 autoantibody.

The present invention offers a clear solution to the problems cited above because it is focused on an *in vitro* method for identifying or screening human subjects at risk of suffering from colorectal cancer or colorectal adenomas (particularly advanced colorectal adenomas), departing from the concentration level of protein biomarkers isolated from minimally-invasive samples such as blood, serum or plasma. Since the method of the invention is based on blood, serum or plasma samples, it is expected to improve compliance to colorectal cancer screening. Moreover, the method of the invention offers high sensitivity and specificity, which means that it is a strong and cost-effective method for the detection of both colorectal cancer and colorectal adenomas.

### DESCRIPTION OF THE INVENTION

The present invention refers to an *in vitro* method for diagnosing, identifying or screening human subjects at risk of suffering from colorectal cancer and/or advanced colorectal adenomas, departing from the concentration level of protein biomarkers isolated from blood, serum or plasma. The method of the invention offers high sensitivity and specificity, which means that it is a strong and cost-effective method for the detection of both colorectal cancer and colorectal adenomas.

Since the method of the invention has higher sensitivity and specificity as compared to the method used today (FIT) for screening general population at risk of suffering from CRC or AA, it is associated with a lower percentage of false positives. Consequently, the method described in the present invention clearly helps in reducing the number of follow-up colonoscopies, thus improving the way that the patients are nowadays screened or diagnosed. Once the method of the invention is performed, if it is determined that the patients might be suffering from colorectal cancer and/or precancerous stage, the result is to be confirmed by colonoscopy. However, if it is not determined that the patient might be suffering from colorectal cancer and/or precancerous stage, there is no need to perform a colonoscopy and routine testing with the method of the invention defined below is recommended.

Particularly, the first embodiment of the present invention refers to an *in vitro* method (hereinafter "method of the invention") according to independent claim 1.

The method of the invention comprises measuring the concentration level of at least the combination [Flt3L and CYFRA21-1] in a biological sample obtained from the subject. Other biomarker signatures that do not comprise this double combination are herein disclosed but not claimed.

In this regard, it is important to consider that all the most reliable signatures claimed in the present invention comprise [Flt3L and CYFRA21-1], thus obtaining various biomarker signatures comprising [Flt3L and CYFRA21-1], such as [Flt3L and CYFRA21-1 and AREG], [Flt3L and CYFRA21-1 and AREG and ErbB4] or [Flt3L and CYFRA21-1 and AREG and CLEC2C] with an Area Under the Curve (AUC) around 0.9 for the detection of CRC and with a good performance also for the detection of AA (see **Table 12**). Although any of the signatures comprising [Flt3L and CYFRA21-1], can be efficiently used according to the present invention, the following signatures comprising [Flt3L and CYFRA21-1], are particularly preferred since they offer an AUC value above 0.9 for the detection of CRC and a good performance for the detection of AA: [Flt3L and CYFRA21-1 and AREG and ErbB4] (AUC for CRC =0.931) or [Flt3L and CYFRA21-1 and AREG and CLEC2C] (AUC for CRC = 0.915) (see **Table 12**).

The second embodiment of the present invention refers to use of a kit according to independent claim 6.

According to the method of the invention, after measuring the concentration level of any of the above cited combinations of biomarkers, a score value is obtained for the signature and this score value is compared with a threshold value which defines the diagnostic rule. If this score value is higher than the threshold, then the corresponding sample is classified as a positive sample, which is an indication that the patient might be suffering from colorectal cancer and/or pre-cancerous stage thereof. The threshold value has been defined in order to optimize sensitivity and specificity values. Consequently, in a preferred embodiment, the method of the invention comprises: a) Measuring the concentration level of any of the above cited combinations of biomarkers, in a biological sample obtained from the subject, b) processing the concentration values in order to obtain a risk score and c) wherein if a deviation or variation of the risk score value obtained for any of the above cited combinations of biomarkers is identified, as compared with a reference value, this is indicative that the subject is suffering from colorectal cancer and/or a pre-cancerous stage.

The third embodiment of the present invention refers to the *in vitro* use of any of the above cited biomarkers or signatures for the diagnosis of colorectal cancer and/or a pre-cancerous stage thereof.

In a preferred embodiment, the pre-cancerous stage of colorectal cancer is advanced colorectal adenoma.

In a preferred embodiment, the diagnosis of the colorectal cancer and/or a pre-cancerous stage thereof is to be confirmed by an image technique, preferably colonoscopy.

In a preferred embodiment the present invention according to the method of the invention further refers to an *in vitro* method for detecting colorectal cancer and/or a precancerous stage thereof, said method comprising: a) obtaining a plasma sample from a human patient; and b) detecting whether any of the above cited protein biomarkers or signatures are present in the plasma sample by contacting the plasma sample with an antibody directed against said protein biomarkers or signatures and detecting binding between the proteins and the antibody.

For the purpose of the present invention the following terms are defined:
- The term "colorectal cancer" is a medical condition characterized by cancer of cells of the intestinal tract below the small intestine (i.e., the large intestine (colon), including the cecum, ascending colon, transverse colon, descending colon, sigmoid colon, and rectum).
- The expression "colorectal adenoma" refers to adenomas of the colon, also called adenomatous polyps, which is a benign and pre-cancerous stage of the colorectal cancer but still with high risk of progression to colorectal cancer.
- The expression "advanced colorectal adenoma" refers to adenomas having a size of at least 10 mm or histologically having high grade dysplasia or a villous component higher than 20%.
- The expression "minimally-invasive biological sample" refers to any sample which is taken from the body of the patient without the need of using harmful instruments, other than fine needles used for taking the blood from the patient, and consequently without being harmfully for the patient. Specifically, minimally-invasive biological sample refers in the present invention to: blood, serum, or plasma samples.
- The expression "reference concentration level measured in healthy control subjects", refer to a "reference value" of the concentration level of the proteins. If a deviation of the concentration level of the proteins is determined with respect to said "reference concentration level measured in healthy control subjects", this is an indication of colorectal cancer or pre-cancerous stage thereof. Particularly, if the concentration level of the biomarkers or signatures of the present invention are significantly higher or lower with respect to said "reference value" this is an indication of colorectal cancer or pre-cancerous stage thereof.
- The expression "risk score" refers to a risk value obtained after processing one or more concentration values into a single value (or risk value), which represents the probability of disease for the individual. This risk value will be compared with a reference value to evaluate if the patient might be suffering from colorectal cancer and/or pre-cancerous stage thereof.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.
- By "comprising" it is meant including, but not limited to, whatever follows the word "comprising". Thus, use of the term "comprising" indicates that the listed elements are required or mandatory, but that other elements are optional and may or may not be present.
- By "consisting of" it is meant "including, and limited to", whatever follows the phrase "consisting of'. Thus, the phrase "consisting of" indicates that the listed elements are required or mandatory, and that no other elements may be present.

For the purpose of the present invention the following proteins are identified according to Uniprot data base:

| **Abbreviation** | **Protein Name** | **Uniprot ID.** |
|---|---|---|
| AREG | Amphiregulin | P15514 |
| CD147 | Basigin | P35613 |
| CLEC2C | Early activation antigen CD69 | Q07108 |
| CYFRA21-1 | Cytokeratin fragment antigen 21-1 | N/A |
| ErbB4 | Receptor tyrosine-protein kinase erbB-4 | Q15303 |
| FasL | Tumor necrosis factor ligand superfamily member 6 | P48023 |
| Flt3L | Fms-related tyrosine kinase 3 ligand | P49771 |
| HGFR | Hepatocyte growth factor receptor | P08581 |
| IFNgamma | Interferon gamma | P01579 |

### Brief description of the figures

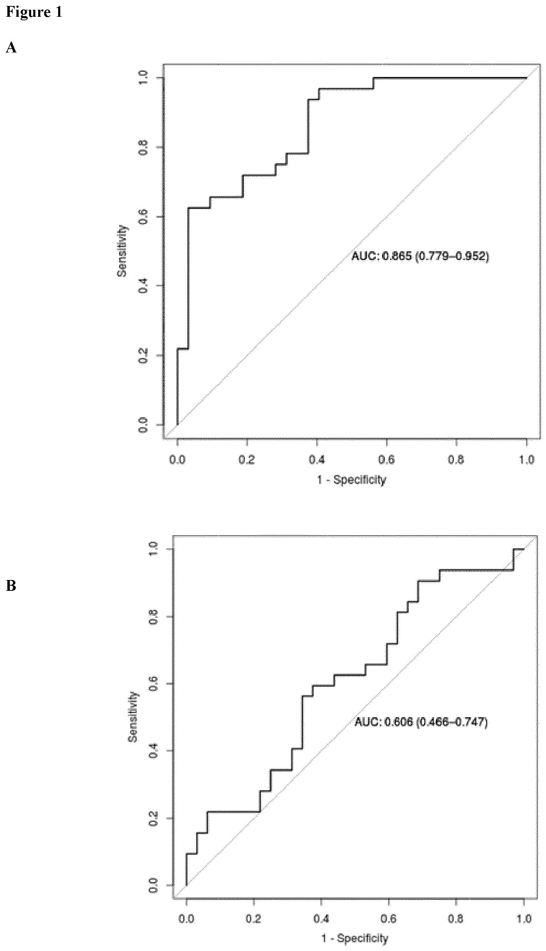

**Figure 1****.** Receiver-operating-characteristic (ROC) curve for: **A)** [Flt3L and CYFRA21-1] in colorectal cancer. Area Under Curve (AUC) = 0.865. **B)** [Flt3L and CYFRA21-1] in advanced colorectal adenoma. Area Under Curve (AUC) = 0.606. X axis represents Specificity. Y axis represents Sensitivity.

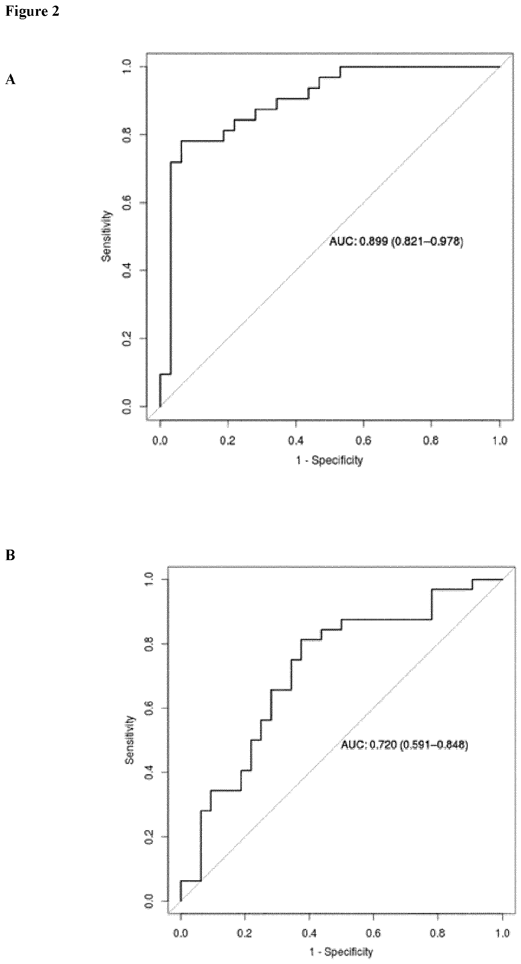

**Figure 2****.** Receiver-operating-characteristic (ROC) curve for: **A)** [Flt3L and CYFRA21-1 and AREG] in colorectal cancer. Area Under Curve (AUC) = 0.899. **B)** [Flt3L and CYFRA21-1 and AREG] in advanced colorectal adenoma. Area Under Curve (AUC) = 0.720. X axis represents Specificity. Y axis represents Sensitivity.

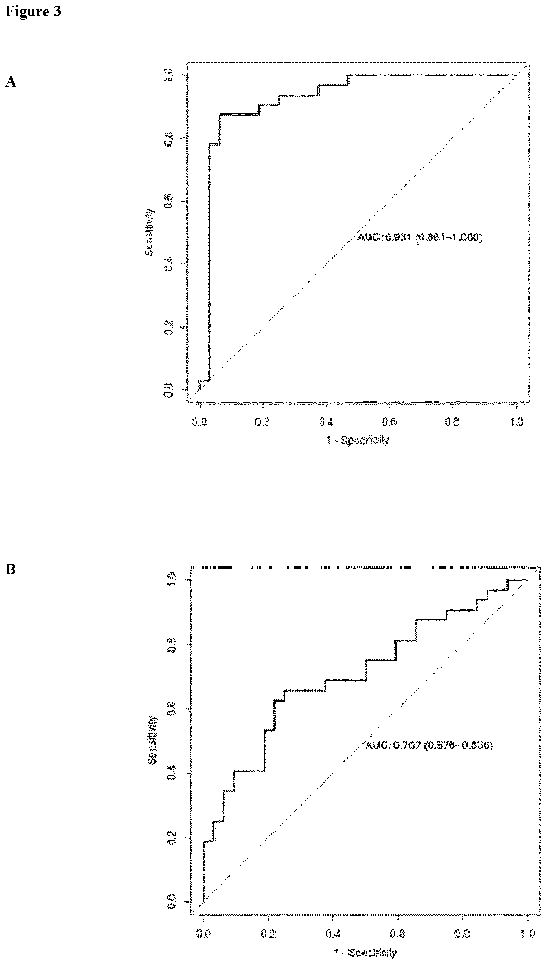

**Figure 3****.** Receiver-operating-characteristic (ROC) curve for: **A)** [Flt3L and CYFRA21-1 and AREG and ErbB4] in colorectal cancer. Area Under Curve (AUC) = 0.931. **B)** [Flt3L and CYFRA21-1 and AREG and ErbB4] in advanced colorectal adenoma. Area Under Curve (AUC) = 0.707. X axis represents Specificity. Y axis represents Sensitivity.

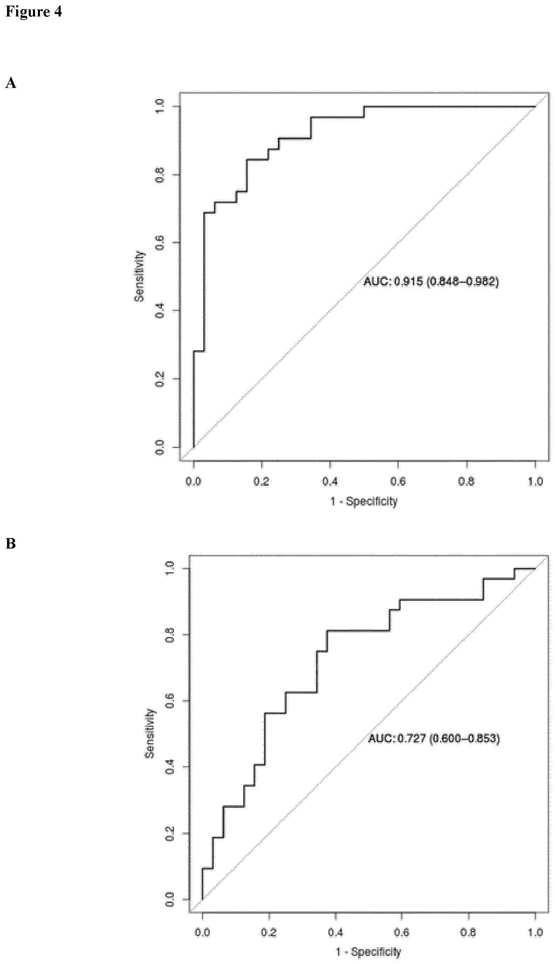

**Figure 4****.** Receiver-operating-characteristic (ROC) curve for: **A)** [Flt3L and CYFRA21-1 and AREG and CLEC2C] in colorectal cancer. Area Under Curve (AUC) = 0.915. **B)** [Flt3L and CYFRA21-1 and AREG and CLEC2C] in advanced colorectal adenoma. Area Under Curve (AUC) = 0.727. X axis represents Specificity. Y axis represents Sensitivity.

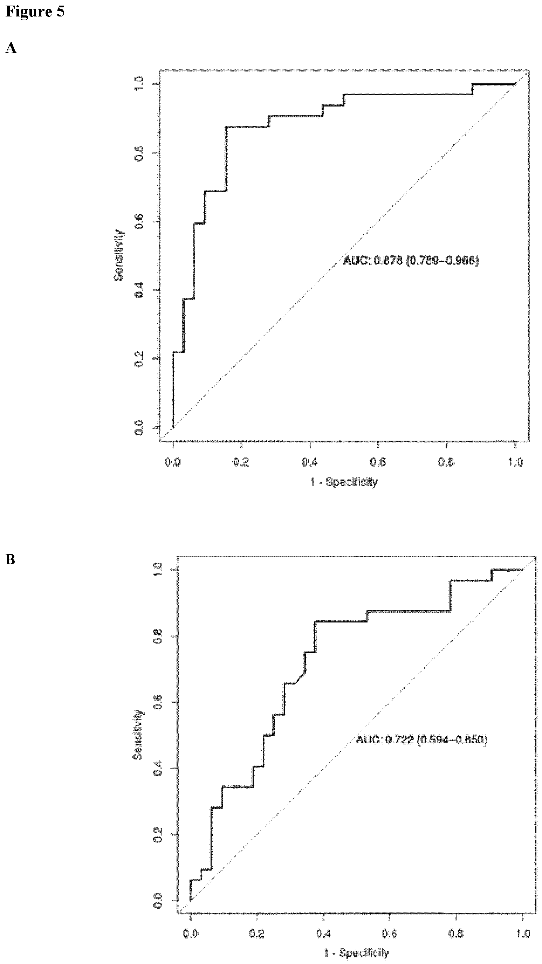

**Figure 5****.** Receiver-operating-characteristic (ROC) curve for: **A)** [AREG and CYFRA21-1] in colorectal cancer. Area Under Curve (AUC) = 0.878. **B)** [AREG and CYFRA21-1] in advanced colorectal adenoma. Area Under Curve (AUC) = 0.722. X axis represents Specificity. Y axis represents Sensitivity.

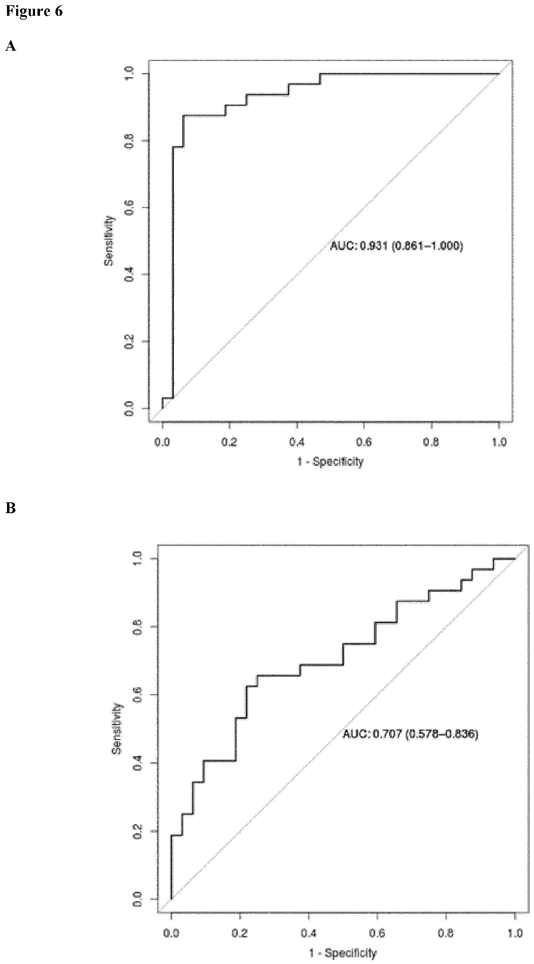

**Figure 6****.** Receiver-operating-characteristic (ROC) curve for: **A)** [AREG and CYFRA21-1 and Flt3L and ErbB4] in colorectal cancer. Area Under Curve (AUC) = 0.931. **B)** [AREG and CYFRA21-1 and Flt3L and ErbB4] in advanced colorectal adenoma. Area Under Curve (AUC) = 0.707. X axis represents Specificity. Y axis represents Sensitivity.

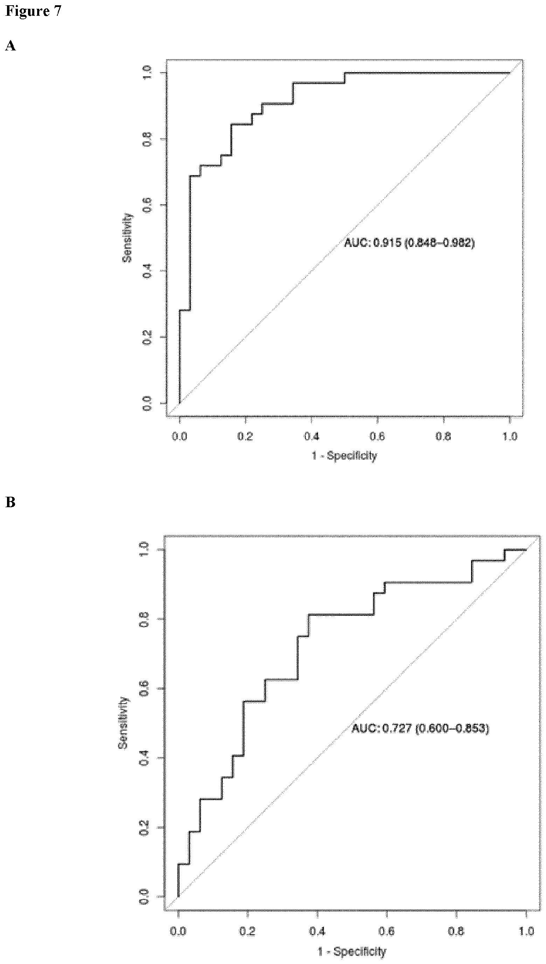

**Figure 7****.** Receiver-operating-characteristic (ROC) curve for: **A)** [AREG and CYFRA21-1 and Flt3L and CLEC2C] in colorectal cancer. Area Under Curve (AUC) = 0.915. **B)** [AREG and CYFRA21-1 and Flt3L and CLEC2C] in advanced colorectal adenoma. Area Under Curve (AUC) = 0.727. X axis represents Specificity. Y axis represents Sensitivity.

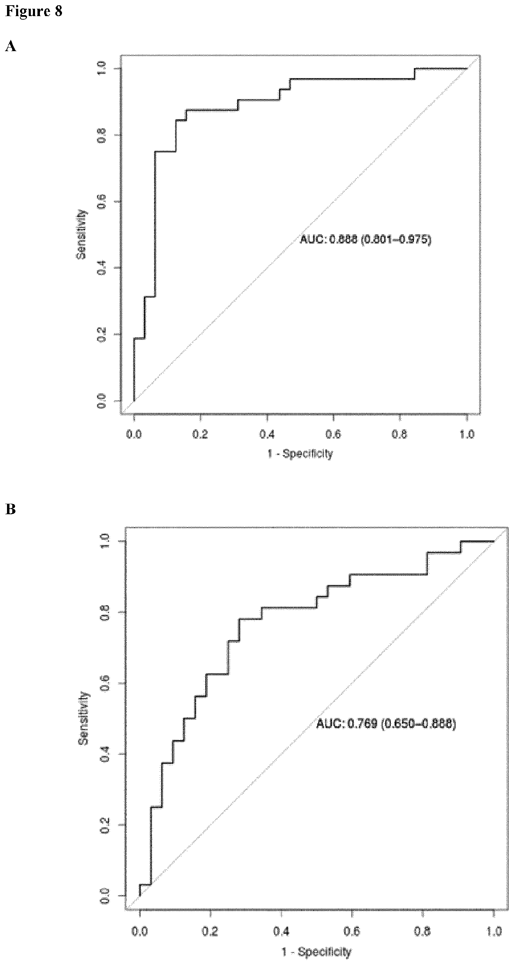

**Figure 8****.** Receiver-operating-characteristic (ROC) curve for: **A)** [AREG and CYFRA21-1 and CD147 and HGFR] in colorectal cancer. Area Under Curve (AUC) = 0.888. **B)** [AREG and CYFRA21-1 and CD147 and HGFR] in advanced colorectal adenoma. Area Under Curve (AUC) = 0.769. X axis represents Specificity. Y axis represents Sensitivity.

### Detailed description of the invention

### Example 1. MATERIAL AND METHODS.

### Example 1.1. Population of study.

A total of 96 subjects from eight Spanish hospitals (Hospital de Burgos, Hospital de Vigo, Hospital de Donosti, Hospital de Ourense, Hospital del Bierzo, Hospital de Beltvigte and Hospital de Zaragoza) were prospectively included in this study: 64 patients newly diagnosed with sporadic colorectal neoplasia (32 with CRC and 32 with AA) and 32 healthy individuals without personal history of any cancer and with a recent colonoscopy confirming the lack of colorectal neoplastic lesions. Patients with AA were those with adenomas having a size of at least 10 mm or histologically having high grade dysplasia or >20% villous component. The characteristics of participants are shown in **Table 1.** Blood samples were collected prior to endoscopy or surgery in all individuals.

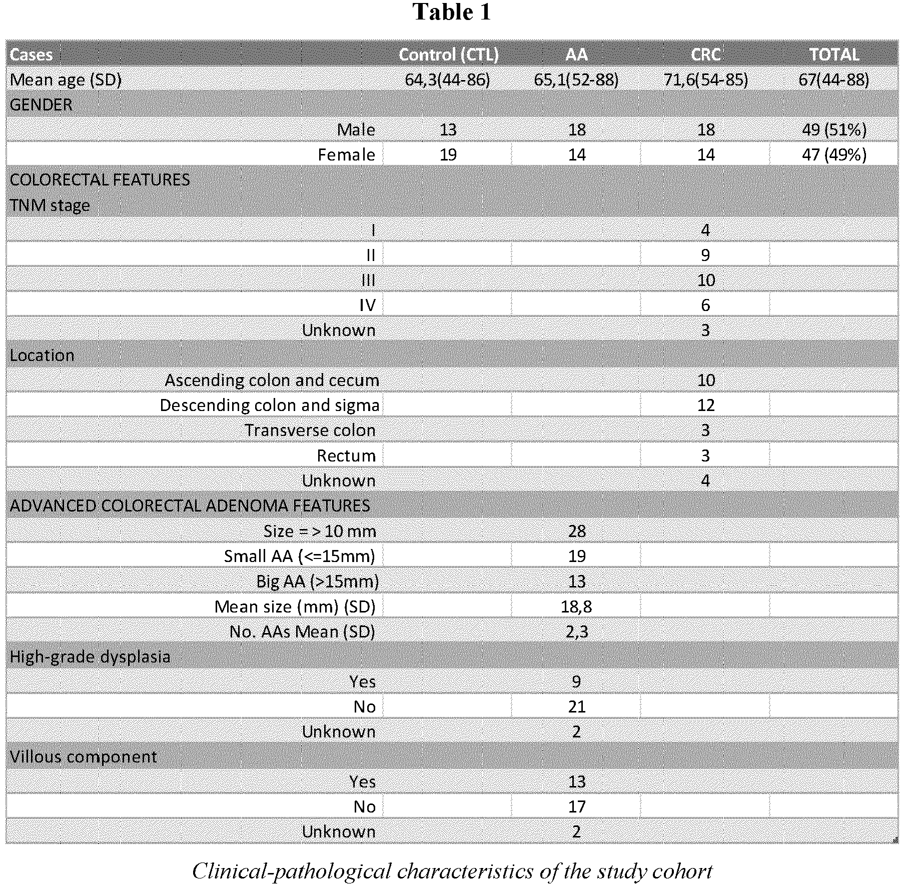

The study was approved by the Institutional Ethics Committee of each Hospital, and written informed consent was obtained from all participants in accordance with the Declaration of Helsinki.

### Example 1.2. Sample preparation.

Ten mL of whole blood from each participant were collected in EDTA K2 containing tubes. Blood samples were placed at 4°C until plasma separation. Samples were centrifuged at 1,600 x g for 10 min at 4°C to spin down blood cells, and plasma was transferred into new tubes, followed by further centrifugation at 16,000 x g for 10 minutes at 4°C to completely remove cellular components.

### Example 1.3. Molecular analysis.

The concentration of the biomarkers in plasma samples was established using commercial ELISA (Enzyme-linked immunosorbent assay) and CLIA (Chemiluminescence immunoassay) test and following their corresponding instruction manual. HGFR and ErbB4 was analyzed with ELISA kit from Cloud clone Corp. Level of CD147, CLEC2C, Flt3L, and FasL was measured using ELISA Kit form Elabscience. In the case of the IFNgamma, ELISA kit from Abcam was used. Related to CLIA test, CYFRA21-1 and AREG were analyzed with CLIA test from Cloud Clone Corp.

### Example 1.4. Data Quantification.

For the protein quantification step the samples were processed with the corresponding kit (ELISA/CLIA) and distributed in experimental plates. Each plate contained also control data used to construct a standard curve. Fluorescence data obtained from each run (expressed as integer numbers) have been background corrected for each sample and quantified using a standard curve generated using a 2-degree polynomial regression model.

### Example 1.5. Statistical analysis.

Three groups of individuals were considered in the analysis. CRC (Individuals diagnosed with colorectal cancer), AA (Individuals diagnosed with advanced adenoma) and CTL (Individuals with no disease).

Raw quantification data have been transformed by applying square root function, and then centering and scaling so that, after the transformation, each protein measure have mean 0 and standard deviation 1. Quantification values are summarized in **Table 2** and **Table 3,** where each protein is described as median and interquartile range in the different groups considered.

Non-normality of the data was confirmed by Shapiro-Wilk test and, consequently, Wilcoxon rank-sum test was used to compare either CRC cases or AA cases against CTL individuals.

Diagnostic performance for the individual proteins and some of their combinations has been assessed by their receiver operating characteristic (ROC) curves, and the area under the ROC curves (AUC). Moreover, sensitivities, specificities, positive predictive and negative predictive values (PPV and NPV) for the different tests were calculated at the optimal cutoff point defined by the best Youden's Index (or equivalently, the point of the ROC that maximizes the sum of sensitivity and specificity).

Scores used for deriving the ROC-AUCs and the rest of performance values were obtained using univariate logistic regression model for the individual proteins and multivariate logistic regression models for the different combination of proteins considered. 95% CI for the AUCs was obtained with the DeLong methodology both in individual markers and combination of them.

### Example 2. RESULTS.

### Example 2.1. Individual marker results.

Different metrics to evaluate the individual proteins were determined, also perming the following comparisons: CRC/AA vs CTL. It can be seen that AREG, CYFRA21-1 and Flt3L are significantly different between CRC and CTL groups and their AUC are significantly different from 0.5 (as their 95% confidence interval do not include 0.5).In the case of AA group, AREG also shows statistically differences compared to CTL group.

**Table 2** and **Table 3** shows metrics for individual proteins, including p-value from Wilcoxon test (p.Wilc), area under the ROC curve (AUC), and Sensitivity (Sens.), Specificity (Spec.), and positive (VPP) and negative (VPN) predictive values computed in the cut-off point of the ROC curve with the best Youden's index. The sign column indicates, for the biomarkers with p-value < 0.25, whether high levels of the marker increase or decrease the risk of disease (+ and - respectively).

**Table 2**

| | **CRC.vs.CTL** | | | | | | |
|---|---|---|---|---|---|---|---|
| | **p.Wilc.** | **Sign** | **AUC** | **Sens.** | **Spec.** | **VPP** | **VPN** |
| **AREG** | 0.0008 | + | 0.744 (0.619,0.868) | 65.62 | 81.25 | 77.78 | 70.27 |
| **CD147** | 0.5280 | | 0.546 (0.402,0.691) | 53.12 | 62.50 | 58.62 | 57.14 |
| **CLEC2C** | 0.2021 | + | 0.593 (0.452,0.734) | 93.75 | 28.12 | 56.60 | 81.82 |
| **CYFRA21-1** | 0.0000 | + | 0.795 (0.682,0.909) | 90.62 | 68.75 | 74.36 | 88.00 |
| **ErbB4** | 0.1177 | - | 0.614 (0.47,0.758) | 53.12 | 75.00 | 68.00 | 61.54 |
| **FasL** | 0.1859 | + | 0.598 (0.454,0.741) | 54.84 | 68.75 | 62.96 | 61.11 |
| **Flt3L** | 0.0191 | - | 0.67 (0.531,0.809) | 56.25 | 84.38 | 78.26 | 65.85 |
| **HGFR** | 0.1729 | + | 0.6 (0.452,0.747) | 65.62 | 68.75 | 67.74 | 66.67 |
| **IFNgamma** | 0.1537 | + | 0.604 (0.464,0.745) | 68.75 | 53.12 | 59.46 | 62.96 |

**Table 3**

| | **AA.vs.CTL** | | | | | | |
|---|---|---|---|---|---|---|---|
| | **p.Wilc.** | **Sign** | **AUC** | **Sens.** | **Spec.** | **VPP** | **VPN** |
| **AREG** | 0.0286 | + | 0.66 (0.525,0.795) | 87.50 | 40.62 | 59.57 | 76.47 |
| **CD147** | 0.1772 | + | 0.599 (0.458,0.739) | 87.50 | 31.25 | 56.00 | 71.43 |
| **CLEC2C** | 0.7831 | | 0.521 (0.376,0.665) | 100.00 | 9.38 | 52.46 | 100.00 |
| **CYFRA21-1** | 0.1412 | + | 0.607 (0.467,0.748) | 90.62 | 37.50 | 59.18 | 80.00 |
| **ErbB4** | 0.3270 | | 0.572 (0.427,0.716) | 50.00 | 71.88 | 64.00 | 58.97 |
| **FasL** | 0.5730 | | 0.542 (0.394,0.689) | 74.19 | 46.88 | 57.50 | 65.22 |
| **Flt3L** | 0.8155 | | 0.518 (0.372,0.663) | 43.75 | 75.00 | 63.64 | 57.14 |
| **HGFR** | 0.0678 | + | 0.633 (0.493,0.773) | 68.75 | 62.50 | 64.71 | 66.67 |
| **IFNgamma** | 0.8467 | | 0.515 (0.37,0.659) | 81.25 | 31.25 | 54.17 | 62.50 |

### Example 2.2. Best combinations of biomarkers.

With the aim of improving individual diagnostic capability, combinations of proteins have been explored with the following procedure: Based on markers with p<0.25 either in CRC.vs.CTL (**Table 3**) or AA.vs.CTL (**Table 4**) comparisons, we used multivariate logistic regression to explore all possible combinations of two, three and four of these proteins taken at the same time.

**Table 4, Table 5, Table 5 bis, Table 6** and **Table 6 bis** show the AUC achieved for the combinations of two, three and four biomarkers respectively, discriminating CRC vs CTL.

**Table 4**

| **Combinations of two biomarkers CRC vs CTL** | **AUC** |
|---|---|
| AREG, CYFRA21-1 | 0.8779297 |
| Flt3L,CYFRA21-1 | 0.8652344 |
| CYFRA21-1,ErbB4 | 0.8359375 |
| AREG,CLEC2C | 0.8349609 |
| CYFRA21-1,IFNgamma | 0.8017578 |
| CYFRA21-1,FasL | 0.7993952 |
| CLEC2C,CYFRA21-1 | 0.7988281 |
| CYFRA21-1,HGFR | 0.7919922 |
| AREG,Flt3L | 0.7832031 |
| AREG,HGFR | 0.7626953 |
| AREG,IFNgamma | 0.7431641 |
| AREG,ErbB4 | 0.7324219 |
| AREG,FasL | 0.7247984 |
| Flt3L,HGFR | 0.6982422 |
| CLEC2C,Flt3L | 0.6953125 |
| ErbB4,Flt3L | 0.6914062 |
| FasL,Flt3L | 0.6814516 |
| Flt3L,IFNgamma | 0.6708984 |
| ErbB4,IFNgamma | 0.6318359 |
| ErbB4,HGFR | 0.6142578 |
| CLEC2C,IFNgamma | 0.6083984 |
| CLEC2C,ErbB4 | 0.6064453 |
| FasL,HGFR | 0.6018145 |
| HGFR,IFNgamma | 0.5869141 |
| CLEC2C,FasL | 0.5866935 |
| ErbB4,FasL | 0.5856855 |
| FasL,IFNgamma | 0.5836694 |
| CLEC2C,HGFR | 0.5810547 |

**Table 5**

| **Combinations of three biomarkers for CRC vs CTL supporting the combination Flt3L+CYFRA21-1** | **AUC** |
|---|---|
| Flt3L,CYFRA21-1,AREG | 0.8994 141 |
| AREG,CLEC2C,CYFRA21-1 | 0.8955 078 |
| AREG, CYFRA21-1 ,ErbB4 | 0.8955 078 |
| AREG,CYFRA21-1,FasL | 0.8931 452 |
| Flt3L,CYFRA21-1 | 0.8847 656 |
| AREG,CYFRA21-1,IFNgamma | 0.8837 891 |
| AREG,CYFRA21-1,HGFR | 0.8759 766 |
| Flt3L,CYFRA21-1,CLEC2C | 0.8681 641 |
| Flt3L,CYFRA21-1,FasL | 0.8679 435 |
| Flt3L,CYFRA21-1,IFNgamma | 0.8671 875 |
| Flt3L,CYFRA21-1,HGFR | 0.8662 109 |
| AREG,CLEC2C,Flt3L | 0.8427 734 |
| CYFRA21-1,ErbB4,HGFR | 0.8388 672 |
| CYFRA21-1,ErbB4,IFNgamma | 0.8359 375 |
| CLEC2C,CYFRA21-1,ErbB4 | 0.8349 609 |
| AREG,CLEC2C,HGFR | 0.8330 078 |
| CYFRA21-1,ErbB4,FasL | 0.8326 613 |
| AREG,CLEC2C,FasL | 0.8316 532 |
| AREG,CLEC2C,ErbB4 | 0.8300 781 |
| AREG,CLEC2C,IFNgamma | 0.8173 828 |
| AREG,Flt3L,HGFR | 0.8066 406 |
| CLEC2C,CYFRA21-1,IFNgamma | 0.8066 406 |
| CYFRA21-1,FasL,IFNgamma | 0.8054 435 |
| CYFRA21-1,HGFR,IFNgamma | 0.8037 109 |
| CYFRA21 -1 ,FasL,HGFR | 0.8014 113 |
| CLEC2C,CYFRA21-1,FasL | 0.7973 790 |
| CLEC2C,CYFRA21-1,HGFR | 0.7968 750 |
| AREG,ErbB4,Flt3L | 0.7841 797 |
| AREG,Flt3L,IFNgamma | 0.7832 031 |
| AREG,FasL,Flt3L | 0.7782 258 |
| AREG,ErbB4,HGFR | 0.7705 078 |
| AREG,HGFR,IFNgamma | 0.7666 016 |
| AREG,FasL,HGFR | 0.7641 129 |
| AREG,ErbB4,IFNgamma | 0.7451 172 |
| AREG,FasL,IFNgamma | 0.7358 871 |
| AREG,ErbB4,FasL | 0.7197 581 |
| ErbB4,Flt3L,HGFR | 0.7119 141 |
| CLEC2C,FasL,Flt3L | 0.7076 613 |
| CLEC2C,ErbB4,Flt3 L | 0.7031 250 |
| ErbB4,FasL,Flt3L | 0.7006 048 |
| CLEC2C,Flt3L,HGFR | 0.6962 891 |
| FasL,Flt3L,HGFR | 0.6935 484 |
| CLEC2C,Flt3L,IFNgamma | 0.6933 594 |
| Flt3L,HGFR,IFNgamma | 0.6894 531 |
| ErbB4,Flt3L,IFNgamma | 0.6875 000 |
| FasL,Flt3L,IFNgamma | 0.6814 516 |
| ErbB4,FasL,IFNgamma | 0.6250 000 |
| ErbB4,HGFR,IFNgamma | 0.6230 469 |
| CLEC2C,ErbB4,IFNgamma | 0.6191 406 |
| ErbB4,FasL,HGFR | 0.6139 113 |
| CLEC2C,ErbB4,HGFR | 0.6123 047 |
| CLEC2C,ErbB4,FasL | 0.6068 548 |
| CLEC2C,FasL,IFNgamma | 0.6048 387 |
| FasL,HGFR,IFNgamma | 0.5997 984 |
| CLEC2C,FasL,HGFR | 0.5967 742 |
| CLEC2C,HGFR,IFNgamma | 0.5966 797 |

**Table 5 bis**

| **Combinations of three biomarkers for CRC vs CTL supporting the combination AREG+CYFRA21-1** | **AUC** |
|---|---|
| AREG,CYFRA21-1,Flt3L | 0.8994 141 |
| AREG,CYFRA21-1,CLEC2C | 0.8955 078 |
| AREG, CYFRA21-1 ,ErbB4 | 0.8955 078 |
| AREG,CYFRA21-1,FasL | 0.8931 452 |
| CYFRA21-1,ErbB4,Flt3L | 0.8847 656 |
| AREG,CYFRA21-1,IFNgamma | 0.8837 891 |
| AREG,CYFRA21-1,HGFR | 0.8759 766 |
| CLEC2C,CYFRA21-1,Flt3L | 0.8681 641 |
| CYFRA21-1,FasL,Flt3L | 0.8679 435 |
| CYFRA21-1,Flt3L,IFNgamma | 0.8671 875 |
| CYFRA21-1,Flt3L,HGFR | 0.8662 109 |
| AREG,CLEC2C,Flt3L | 0.8427 734 |
| CYFRA21-1,ErbB4,HGFR | 0.8388 672 |
| CYFRA21-1,ErbB4,IFNgamma | 0.8359 375 |
| CLEC2C,CYFRA21-1,ErbB4 | 0.8349 609 |
| AREG,CLEC2C,HGFR | 0.8330 078 |
| CYFRA21-1,ErbB4,FasL | 0.8326 613 |
| AREG,CLEC2C,FasL | 0.8316 532 |
| AREG,CLEC2C,ErbB4 | 0.8300 781 |
| AREG,CLEC2C,IFNgamma | 0.8173 828 |
| AREG,Flt3L,HGFR | 0.8066 406 |
| CLEC2C,CYFRA21-1,IFNgamma | 0.8066 406 |
| CYFRA21-1,FasL,IFNgamma | 0.8054 435 |
| CYFRA21-1,HGFR,IFNgamma | 0.8037 109 |
| CYFRA21-1,FasL,HGFR | 0.8014 113 |
| CLEC2C,CYFRA21-1,FasL | 0.7973 790 |
| CLEC2C,CYFRA21-1,HGFR | 0.7968 750 |
| AREG,ErbB4,Flt3L | 0.7841 797 |
| AREG,Flt3L,IFNgamma | 0.7832 031 |
| AREG,FasL,Flt3L | 0.7782 258 |
| AREG,ErbB4,HGFR | 0.7705 078 |
| AREG,HGFR,IFNgamma | 0.7666 016 |
| AREG,FasL,HGFR | 0.7641 129 |
| AREG,ErbB4,IFNgamma | 0.7451 172 |
| AREG,FasL,IFNgamma | 0.7358 871 |
| AREG,ErbB4,FasL | 0.7197 581 |
| ErbB4,Flt3L,HGFR | 0.7119 141 |
| CLEC2C,FasL,Flt3L | 0.7076 613 |
| CLEC2C,ErbB4,Flt3 L | 0.7031 250 |
| ErbB4,FasL,Flt3L | 0.7006 048 |
| CLEC2C,Flt3L,HGFR | 0.6962 891 |
| FasL,Flt3L,HGFR | 0.6935 484 |
| CLEC2C,Flt3L,IFNgamma | 0.6933 594 |
| Flt3L,HGFR,IFNgamma | 0.6894 531 |
| ErbB4,Flt3L,IFNgamma | 0.6875 000 |
| FasL,Flt3L,IFNgamma | 0.6814 516 |
| ErbB4,FasL,IFNgamma | 0.6250 000 |
| ErbB4,HGFR,IFNgamma | 0.6230 469 |
| CLEC2C,ErbB4,IFNgamma | 0.6191 406 |
| ErbB4,FasL,HGFR | 0.6139 113 |
| CLEC2C,ErbB4,HGFR | 0.6123 047 |
| CLEC2C,ErbB4,FasL | 0.6068 548 |
| CLEC2C,FasL,IFNgamma | 0.6048 387 |
| FasL,HGFR,IFNgamma | 0.5997 984 |
| CLEC2C,FasL,HGFR | 0.5967 742 |
| CLEC2C,HGFR,IFNgamma | 0.5966 797 |

**Table 6**

| **Combinations of four biomarkers for CRC vs CTL supporting the combination Flt3L+CYFRA21-1** | **AUC** |
|---|---|
| Flt3L,CYFRA21-1,ErbB4,AREG | 0.9306 641 |
| Flt3L,CYFRA21-1,AREG,CLEC2C | 0.9150 391 |
| AREG,CLEC2C,CYFRA21-1,ErbB4 | 0.9023 438 |
| AREG,CYFRA21-1,ErbB4,FasL | 0.9022 177 |
| AREG,CLEC2C,CYFRA21-1,IFNgamma | 0.9003 906 |
| Flt3L,CYFRA21-1,HGFR,AREG, | 0.9003 906 |
| AREG,CLEC2C,CYFRA21-1,FasL | 0.9002 016 |
| AREG,CYFRA21-1,ErbB4,IFNgamma | 0.8994 141 |
| Flt3L,CYFRA21-1,IFNgamma,AREG | 0.8984 375 |
| Flt3L,CYFRA21-1,FasL,AREG | 0.8981 855 |
| AREG,CYFRA21-1,FasL,IFNgamma | 0.8951 613 |
| AREG,CYFRA21-1,ErbB4,HGFR | 0.8945 312 |
| AREG,CLEC2C,CYFRA21-1,HGFR | 0.8935 547 |
| AREG,CYFRA21-1,FasL,HGFR | 0.8901 210 |
| Flt3L,CYFRA21-1,ErbB4,HGFR | 0.8867 188 |
| Flt3L,CYFRA21-1,ErbB4,IFNgamma | 0.8867 188 |
| AREG,CYFRA21-1,HGFR,IFNgamma | 0.8847 656 |
| Flt3L,CYFRA21-1,ErbB4,CLEC2C | 0.8847 656 |
| Flt3L,CYFRA21-1,ErbB4,FasL | 0.8830 645 |
| Flt3L,CYFRA21-1,FasL,IFNgamma | 0.8689 516 |
| Flt3L,CYFRA21-1,HGFR,IFNgamma | 0.8671 875 |
| Flt3L,CYFRA21-1,FasL,HGFR | 0.8669 355 |
| Flt3L,CYFRA21-1,IFNgamma,CLEC2C | 0.8662 109 |
| Flt3L,CYFRA21-1,FasL,CLEC2C | 0.8649 194 |
| Flt3L,CYFRA21-1,HGFR,CLEC2C | 0.8632 812 |
| AREG,CLEC2C,Flt3L,HGFR | 0.8564 453 |
| AREG,CLEC2C,ErbB4,Flt3L | 0.8457 031 |
| AREG,CLEC2C,FasL,Flt3L | 0.8447 581 |
| AREG,CLEC2C,Flt3L,IFNgamma | 0.8447 266 |
| CYFRA21-1,ErbB4,HGFR,IFNgamma | 0.8417 969 |
| CLEC2C,CYFRA21-1,ErbB4,HGFR | 0.8398 438 |
| AREG,CLEC2C,ErbB4,HGFR | 0.8378 906 |
| CLEC2C,CYFRA21-1,ErbB4,IFNgamma | 0.8359 375 |
| AREG,CLEC2C,ErbB4,FasL | 0.8346 774 |
| AREG,CLEC2C,FasL,HGFR | 0.8326 613 |
| CLEC2C,CYFRA21-1,ErbB4,FasL | 0.8326 613 |
| CYFRA21-1,ErbB4,FasL,IFNgamma | 0.8326 613 |
| CYFRA21-1,ErbB4,FasL,HGFR | 0.8316 532 |
| AREG,CLEC2C,HGFR,IFNgamma | 0.8271 484 |
| AREG,CLEC2C,ErbB4,IFNgamma | 0.8251 953 |
| AREG,CLEC2C,FasL,IFNgamma | 0.8245 968 |
| AREG,ErbB4,Flt3L,HGFR | 0.8203 125 |
| CLEC2C,CYFRA21-1,FasL,IFNgamma | 0.8074 597 |
| AREG,Flt3L,HGFR,IFNgamma | 0.8066 406 |
| CYFRA21-1,FasL,HGFR,IFNgamma | 0.8064 516 |
| CLEC2C,CYFRA21-1,HGFR,IFNgamma | 0.8056 641 |
| AREG,FasL,Flt3L,HGFR | 0.7993 952 |
| CLEC2C, CYFRA21-1 ,FasL,HGFR | 0.7983 871 |
| AREG,ErbB4,Flt3L,IFNgamma | 0.7861 328 |
| AREG,ErbB4,HGFR,IFNgamma | 0.7802 734 |
| AREG,ErbB4,FasL,Flt3L | 0.7802 419 |
| AREG,FasL,Flt3L,IFNgamma | 0.7772 177 |
| AREG,ErbB4,FasL,HGFR | 0.7721 774 |
| AREG,FasL,HGFR,IFNgamma | 0.7681 452 |
| AREG,ErbB4,FasL,IFNgamma | 0.7419 355 |
| ErbB4,FasL,Flt3L,HGFR | 0.7358 871 |
| CLEC2C,ErbB4,FasL,Flt3L | 0.7167 339 |
| CLEC2C,FasL,Flt3L,HGFR | 0.7086 694 |
| CLEC2C,FasL,Flt3L,IFNgamma | 0.7056 452 |
| CLEC2C,ErbB4,Flt3L,HGFR | 0.7050 781 |
| ErbB4,Flt3L,HGFR,IFNgamma | 0.7050 781 |
| CLEC2C,ErbB4,Flt3L,IFNgamma | 0.7011 719 |
| ErbB4,FasL,Flt3L,IFNgamma | 0.6985 887 |
| CLEC2C,Flt3L,HGFR,IFNgamma | 0.6933 594 |
| FasL,Flt3L,HGFR,IFNgamma | 0.6895 161 |
| CLEC2C,ErbB4,FasL,IFNgamma | 0.6290 323 |
| CLEC2C,ErbB4,FasL,HGFR | 0.6239 919 |
| CLEC2C,ErbB4,HGFR,IFNgamma | 0.6220 703 |
| ErbB4,FasL,HGFR,IFNgamma | 0.6118 952 |
| CLEC2C,FasL,HGFR,IFNgamma | 0.6088 710 |

**Table 6 bis**

| **Combinations of four biomarkers for CRC vs CTL supporting the combination AREG+CYFRA21-1** | **AUC** |
|---|---|
| AREG,CYFRA21-1, Flt3L, ErbB4 | 0.9306 641 |
| AREG,CYFRA21-1, Flt3L CLEC2C | 0.9150 391 |
| AREG,CYFRA21-1,ErbB4, CLEC2C | 0.9023 438 |
| AREG,CYFRA21-1,ErbB4,FasL | 0.9022 177 |
| AREG,CYFRA21-1,CLEC2C,IFNgamma | 0.9003 906 |
| AREG,CYFRA21-1,Flt3L,HGFR | 0.9003 906 |
| AREG,CYFRA21-1,CLEC2C,FasL | 0.9002 016 |
| AREG,CYFRA21-1,ErbB4,IFNgamma | 0.8994 141 |
| AREG, CYFRA21-1,Flt3L,IFNgamma | 0.8984 375 |
| AREG,CYFRA21-1, Flt3L, FasL, | 0.8981 855 |
| AREG,CYFRA21-1,FasL,IFNgamma | 0.8951 613 |
| AREG,CYFRA21-1,ErbB4,HGFR | 0.8945 312 |
| AREG,CYFRA21-1,HGFR,CLEC2C, | 0.8935 547 |
| AREG,CYFRA21-1,FasL,HGFR | 0.8901 210 |
| CYFRA21-1,ErbB4,Flt3L,HGFR | 0.8867 188 |
| CYFRA21-1,ErbB4,Flt3L,IFNgamma | 0.8867 188 |
| AREG,CYFRA21-1,HGFR,IFNgamma | 0.8847 656 |
| CLEC2C,CYFRA21-1,ErbB4,Flt3L | 0.8847 656 |
| CYFRA21 -1 ,ErbB4,FasL,Flt3L | 0.8830 645 |
| CYFRA21-1,FasL,Flt3L,IFNgamma | 0.8689 516 |
| CYFRA21-1,Flt3L,HGFR,IFNgamma | 0.8671 875 |
| CYFRA21-1,FasL,Flt3L,HGFR | 0.8669 355 |
| CLEC2C,CYFRA21-1,Flt3L,IFNgamma | 0.8662 109 |
| CLEC2C,CYFRA21-1,FasL,Flt3L | 0.8649 194 |
| CLEC2C,CYFRA21-1,Flt3L,HGFR | 0.8632 812 |
| AREG,CLEC2C,Flt3L,HGFR | 0.8564 453 |
| AREG,CLEC2C,ErbB4,Flt3L | 0.8457 031 |
| AREG,CLEC2C,FasL,Flt3L | 0.8447 581 |
| AREG,CLEC2C,Flt3L,IFNgamma | 0.8447 266 |
| CYFRA21-1,ErbB4,HGFR,IFNgamma | 0.8417 969 |
| CLEC2C,CYFRA21-1,ErbB4,HGFR | 0.8398 438 |
| AREG,CLEC2C,ErbB4,HGFR | 0.8378 906 |
| CLEC2C,CYFRA21-1,ErbB4,IFNgamma | 0.8359 375 |
| AREG,CLEC2C,ErbB4,FasL | 0.8346 774 |
| AREG,CLEC2C,FasL,HGFR | 0.8326 613 |
| CLEC2C,CYFRA21-1,ErbB4,FasL | 0.8326 613 |
| CYFRA21-1,ErbB4,FasL,IFNgamma | 0.8326 613 |
| CYFRA21-1,ErbB4,FasL,HGFR | 0.8316 532 |
| AREG,CLEC2C,HGFR,IFNgamma | 0.8271 484 |
| AREG,CLEC2C,ErbB4,IFNgamma | 0.8251 953 |
| AREG,CLEC2C,FasL,IFNgamma | 0.8245 968 |
| AREG,ErbB4,Flt3L,HGFR | 0.8203 125 |
| CLEC2C,CYFRA21-1,FasL,IFNgamma | 0.8074 597 |
| AREG,Flt3L,HGFR,IFNgamma | 0.8066 406 |
| CYFRA21-1,FasL,HGFR,IFNgamma | 0.8064 516 |
| CLEC2C,CYFRA21-1,HGFR,IFNgamma | 0.8056 641 |
| AREG,FasL,Flt3L,HGFR | 0.7993 952 |
| CLEC2C,CYFRA21-1,FasL,HGFR | 0.7983 871 |
| AREG,ErbB4,Flt3L,IFNgamma | 0.7861 328 |
| AREG,ErbB4,HGFR,IFNgamma | 0.7802 734 |
| AREG,ErbB4,FasL,Flt3L | 0.7802 419 |
| AREG,FasL,Flt3L,IFNgamma | 0.7772 177 |
| AREG,ErbB4,FasL,HGFR | 0.7721 774 |
| AREG,FasL,HGFR,IFNgamma | 0.7681 452 |
| AREG,ErbB4,FasL,IFNgamma | 0.7419 355 |
| ErbB4,FasL,Flt3L,HGFR | 0.7358 871 |
| CLEC2C,ErbB4,FasL,Flt3L | 0.7167 339 |
| CLEC2C,FasL,Flt3L,HGFR | 0.7086 694 |
| CLEC2C,FasL,Flt3L,IFNgamma | 0.7056 452 |
| CLEC2C,ErbB4,Flt3L,HGFR | 0.7050 781 |
| ErbB4,Flt3L,HGFR,IFNgamma | 0.7050 781 |
| CLEC2C,ErbB4,Flt3L,IFNgamma | 0.7011 719 |
| ErbB4,FasL,Flt3L,IFNgamma | 0.6985 887 |
| CLEC2C,Flt3L,HGFR,IFNgamma | 0.6933 594 |
| FasL,Flt3L,HGFR,IFNgamma | 0.6895 161 |
| CLEC2C,ErbB4,FasL,IFNgamma | 0.6290 323 |
| CLEC2C,ErbB4,FasL,HGFR | 0.6239 919 |
| CLEC2C,ErbB4,HGFR,IFNgamma | 0.6220 703 |
| ErbB4,FasL,HGFR,IFNgamma | 0.6118 952 |
| CLEC2C,FasL,HGFR,IFNgamma | 0.6088 710 |

**Table 7, Table 8** and **Table 9** show the AUC achieved for the combinations of two, three and four biomarkers respectively, discriminating AA vs CTL.

**Table 7**

| **Biomarker combination AA vs CTL** | **AUC** |
|---|---|
| AREG,CD 147 | 0.7548828 |
| AREG,HGFR | 0.7460938 |
| AREG,CYFRA21-1 | 0.7221680 |
| CYFRA21-1,HGFR | 0.6250000 |
| CD147,HGFR | 0.6191406 |
| CD147,CYFRA21-1 | 0.6152344 |

**Table 8**

| **Biomarker combination AA vs CTL** | **AUC** |
|---|---|
| AREG,CD147,CYFRA21-1 | 0.7617188 |
| AREG,CYFRA21-1,HGFR | 0.7607422 |
| AREG,CD147,HGFR | 0.7539062 |
| CD147,CYFRA21-1,HGFR | 0.6337891 |

**Table 9**

| **Biomarker combination AA vs CTL** | **AUC** |
|---|---|
| AREG,CYFRA21-1,HGFR,CD147 | 0.7685547 |

Based on their respective AUCs, the best models have been selected. **Table 10** and **Table 10bis** show the best results for CRC. **Table 11** and **Table 11bis** show the best results for AA. The metrics for the best combinations of proteins are included, comprising area under the ROC curve (AUC), Sensitivity (Sens.), Specificity (Spec.), and positive (PPV) and negative (NPV) predictive values computed in the cut-off point of the ROC curve with the best Youden's index.

**Table 10**

| | **CRC.vs.CTL** | | | | |
|---|---|---|---|---|---|
| **Biomarker combination supporting Flt3L+CYFRA21-1** | **AUC (95%CI)** | **Sen s** | **Spe c** | **PP V** | **NP V** |
| Flt3L,CYFRA21-1 | 0.865(0.779,0.9 52) | 0.62 5 | 0.96 9 | 0.95 2 | 0.72 1 |
| Flt3L,CYFRA21-1,AREG | 0.899(0.821,0.9 78) | 0.78 1 | 0.93 8 | 0.92 6 | 0.81 1 |
| Flt3L,CYFRA21-1,AREG,ErbB4 | 0.931(0.861,1) | 0.87 5 | 0.93 8 | 0.93 3 | 0.88 2 |
| Flt3L,CYFRA21-1,AREG,CLEC2C | 0.915(0.848,0.9 82) | 0.84 4 | 0.84 4 | 0.84 4 | 0.84 4 |

**Table 10bis**

| | **CRC.vs.CTL** | | | | |
|---|---|---|---|---|---|
| **Biomarker combination supporting AREG+CYFRA21-1** | **AUC (95%CI)** | **Sen s** | **Spe c** | **PP V** | **NP V** |
| AREG,CYFRA21-1 | 0.878(0.789,0. 966) | 0.87 5 | 0.84 4 | 0.84 8 | 0.87 1 |
| AREG,CLEC2C | 0.835(0.729,0. 941) | 0.90 6 | 0.81 2 | 0.82 9 | 0.89 7 |
| AREG,HGFR | 0.763(0.643,0. 883) | 0.65 6 | 0.81 2 | 0.77 8 | 0.70 3 |
| AREG,CD 147 | 0.842(0.734,0. 95) | 0.84 4 | 0.84 4 | 0.84 4 | 0.84 4 |
| AREG,CYFRA21-1,Flt3L | 0.899(0.821,0. 978) | 0.78 1 | 0.93 8 | 0.92 6 | 0.81 1 |
| AREG,CYFRA21-1,CLEC2C | 0.896(0.814,0. 977) | 0.87 5 | 0.84 4 | 0.84 8 | 0.87 1 |
| AREG, CYFRA21-1 ,ErbB4 | 0.896(0.812,0. 979) | 0.87 5 | 0.84 4 | 0.84 8 | 0.87 1 |
| AREG,CYFRA21-1,FasL | 0.893(0.81,0.9 76) | 0.90 3 | 0.81 2 | 0.82 4 | 0.89 7 |
| AREG,CYFRA21-1,CD147 | 0.886(0.797,0. 975) | 0.87 5 | 0.84 4 | 0.84 8 | 0.87 1 |
| AREG,CYFRA21-1,HGFR | 0.876(0.787,0. 965) | 0.87 5 | 0.84 4 | 0.84 8 | 0.87 1 |
| AREG,CD147,HGFR | 0.843(0.735,0. 951) | 0.84 4 | 0.84 4 | 0.84 4 | 0.84 4 |
| AREG,CYFRA21-1,ErbB4,Flt3L | 0.931(0.861,1) | 0.87 5 | 0.93 8 | 0.93 3 | 0.88 2 |
| AREG,CYFRA21-1,Flt3L, CLEC2C | 0.915(0.848,0. 982) | 0.84 4 | 0.84 4 | 0.84 4 | 0.84 4 |
| AREG,CYFRA21-1,CD147,HGFR | 0.888(0.801,0. 975) | 0.87 5 | 0.84 4 | 0.84 8 | 0.87 1 |

**Table 11**

| | | **AA.vs.CTL** | | | |
|---|---|---|---|---|---|
| **Biomarker combination supporting Flt3L+CYFRA21-1** | **AUC (95%CI)** | **Sen s** | **Spe c** | **PP V** | **NP V** |
| Flt3L,CYFRA21-1 | 0.606(0.466,0.7 47) | 0.90 6 | 0.31 2 | 0.56 9 | 0.76 9 |
| Flt3L,CYFRA21-1,AREG | 0.72(0.591,0.84 8) | 0.81 2 | 0.62 5 | 0.68 4 | 0.76 9 |
| Flt3L,CYFRA21 -1 ,AREG,ErbB4 | 0.707(0.578,0.8 36) | 0.65 6 | 0.75 | 0.72 4 | 0.68 6 |
| Flt3L,CYFRA21-1,AREG,CLEC2C | 0.727(0.6,0.853 ) | 0.81 2 | 0.62 5 | 0.68 4 | 0.76 9 |

**Table 11bis**

| | | **AA.vs.CTL** | | | |
|---|---|---|---|---|---|
| **Biomarker combination supporting AREG+CYFRA21-1** | **AUC (95%CI)** | **Sen s** | **Spe c** | **PP V** | **NP V** |
| AREG,CYFRA21-1 | 0.722(0.594,0. 85) | 0.84 4 | 0.62 5 | 0.69 2 | 0.8 |
| AREG,CLEC2C | 0.738(0.613,0. 864) | 0.78 1 | 0.68 8 | 0.71 4 | 0.75 9 |
| AREG,HGFR | 0.746(0.621,0. 871) | 0.53 1 | 0.93 8 | 0.89 5 | 0.66 7 |
| AREG,CD 147 | 0.755(0.633,0. 877) | 0.65 6 | 0.84 4 | 0.80 8 | 0.71 1 |
| AREG,CYFRA21-1,Flt3L | 0.72(0.591,0.8 48) | 0.81 2 | 0.62 5 | 0.68 4 | 0.76 9 |
| AREG,CYFRA21-1,CLEC2C | 0.728(0.601,0. 854) | 0.81 2 | 0.62 5 | 0.68 4 | 0.76 9 |
| AREG,CYFRA21-1,ErbB4 | 0.71(0.582,0.8 38) | 0.65 6 | 0.75 | 0.72 4 | 0.68 6 |
| AREG,CYFRA21-1,FasL | 0.711(0.581,0. 84) | 0.77 4 | 0.62 5 | 0.66 7 | 0.74 1 |
| AREG,CYFRA21-1,CD147 | 0.762(0.642,0. 882) | 0.84 4 | 0.65 6 | 0.71 1 | 0.80 8 |
| AREG,CYFRA21-1,HGFR | 0.761(0.634,0. 887) | 0.75 | 0.75 | 0.75 | 0.75 |
| AREG,CD147,HGFR | 0.754(0.632,0. 876) | 0.56 2 | 0.90 6 | 0.85 7 | 0.67 4 |
| AREG,CYFRA21-1, Flt3L, ErbB4, | 0.707(0.578,0. 836) | 0.65 6 | 0.75 | 0.72 4 | 0.68 6 |
| AREG,CYFRA21-1, Flt3L, CLEC2C | 0.727(0.6,0.85 3) | 0.81 2 | 0.62 5 | 0.68 4 | 0.76 9 |
| AREG,CYFRA21-1,CD147,HGFR | 0.769(0.65,0.8 88) | 0.78 1 | 0.71 9 | 0.73 5 | 0.76 7 |

Finally, **Table 12** and **Table 12bis** have been designed to show the overlapping of the most important signatures claimed in the present invention. It is clearly shown that all the best signature signatures comprise [Flt3L and CYFRA21-1] and [AREG and CYFRA21-1].

**Table 12**

| **AREG** | **CLEC2C** | **CYFRA21-1** | **Flt3L** | **ErbB4** | **CRC vs CTL** | **AA vs CTL** |
|---|---|---|---|---|---|---|
| X | | **X** | **X** | X | **0.931** | 0.707 |
| X | X | **X** | **X** | | 0.915 | **0.727** |
| X | | **X** | **X** | | 0.899 | 0.720 |
| | | **X** | **X** | | 0.865 | 0.606 |

**Table 12 bis**

| **CD147** | **FasL** | **CLEC2C** | **AREG** | **CYFRA21-1** | **Flt3L** | **ErbB4** | **HGFR** | **CRC** | **AA** |
|---|---|---|---|---|---|---|---|---|---|
| | | | **X** | **X** | X | X | | **0.931** | 0.707 |
| | | X | **X** | **X** | X | | | 0.915 | 0.727 |
| | | | **X** | **X** | X | | | 0.899 | 0.720 |
| | | X | **X** | **X** | | | | 0.896 | 0.728 |
| | | | **X** | **X** | | X | | 0.896 | 0.710 |
| | X | | **X** | **X** | | | | 0.893 | 0.711 |
| X | | | **X** | **X** | | | X | 0.888 | **0.769** |
| X | | | **X** | **X** | | | | 0.886 | 0.762 |
| | | | **X** | **X** | | | | 0.878 | 0.722 |
| | | | **X** | **X** | | | X | 0.876 | 0.761 |
| X | | | X | | | | X | 0.843 | 0.754 |
| X | | | X | | | | | 0.842 | 0.755 |
| | | X | X | | | | | 0.835 | 0.738 |
| | | | X | | | | X | 0.763 | 0.746 |

## Claims

1. *In vitro* method for the diagnosis of colorectal cancer and/or a pre-cancerous stage thereof which comprises: a) Measuring the concentration level of the combination [Flt3L and CYFRA21-1] in a biological sample obtained from the subject selected from blood, serum or plasma; and b) wherein if a deviation or variation of the concentration level of at least the combination [Flt3L and CYFRA21-1] is identified, as compared with the reference concentration level measured in healthy control subjects, this is indicative that the subject is suffering from colorectal cancer and/or a pre-cancerous stage.

2. *In vitro* method for the diagnosis of colorectal cancer and/or a pre-cancerous stage thereof, according to claim 1, which comprises: a) Measuring the concentration level of at least the combination [Flt3L and CYFRA21-1 and AREG], or the combination [Flt3L and CYFRA21-1 and AREG and ErbB4] or the combination [Flt3L and CYFRA21-1 and AREG and CLEC2C] in a biological sample obtained from the subject selected from blood, serum or plasma; and b) wherein if a deviation or variation of the concentration level of at least one combination cited in step a) is identified, as compared with the reference concentration level measured in healthy control subjects, this is indicative that the subject is suffering from colorectal cancer and/or a pre-cancerous stage.

3. *In vitro* method, according to any of the previous claims, wherein the pre-cancerous stage of colorectal cancer is advanced colorectal adenoma.

4. *In vitro* use of the combination [Flt3L and CYFRA21-1] in a biological sample obtained from the subject selected from blood, serum or plasma for the diagnosis of colorectal cancer and/or a pre-cancerous stage thereof.

5. *In vitro* use of the combination [Flt3L and CYFRA21-1 and AREG], or [Flt3L and CYFRA21-1 and AREG and ErbB4], or [Flt3L and CYFRA21-1 and AREG and CLEC2C] in a biological sample obtained from the subject selected from blood, serum or plasma, according to claim 4, for the diagnosis of colorectal cancer and/or a pre-cancerous stage thereof.

6. *In vitro* use of a kit comprising reagents adapted for the determination of the concentration
level of at least a signature selected from the list: [Flt3L and CYFRA21-1], or [Flt3L and CYFRA21-1 and AREG], or [Flt3L and CYFRA21-1 and AREG and ErbB4], or [Flt3L and CYFRA21-1 and AREG and CLEC2C] in a biological sample obtained from the subject selected from blood, serum or plasma for the diagnosis of colorectal cancer and/or a pre-cancerous stage thereof.

7. *In vitro* use, according to claim 6, wherein the pre-cancerous stage of colorectal cancer is advanced colorectal adenoma.

## Patentansprüche

1. *In-vitro*-Verfahren zur Diagnose von kolorektalem Krebs und/oder einem präkanzerogenen Stadium desselben, welches Folgendes umfasst: a) das Messen des Konzentrationsniveaus der Kombination [Flt3L und CYFRA21-1] in einer vom Individuum erhaltenen biologischen Probe ausgewählt aus Blut, Serum oder Plasma; und b) wobei, wenn eine Abweichung oder Veränderung des Konzentrationsniveaus von mindestens der Kombination [Flt3L und CYFRA21-1] im Vergleich zum Referenz-Konzentrationsniveau identifiziert wird, welches in gesunden Kontrollindividuen gemessen wird, dies darauf schließen lässt, dass das Individuum unter kolorektalem Krebs und/oder einem präkanzerogenen Stadium desselben leidet.

2. *In-vitro*-Verfahren zur Diagnose von kolorektalem Krebs und/oder einem präkanzerogenen Stadium desselben nach Anspruch 1, welches Folgendes umfasst: a) das Messen des Konzentrationsniveaus von mindestens der Kombination [Flt3L und CYFRA21-1 und AREG] oder der Kombination [Flt3L und CYFRA21-1 und AREG und ErbB4] oder der Kombination [Flt3L und CYFRA21-1 und AREG und CLEC2C] in einer vom Individuum erhaltenen biologischen Probe ausgewählt aus Blut, Serum oder Plasma; und b) wobei, wenn eine Abweichung oder Veränderung des Konzentrationsniveaus von mindestens einer in Schritt a) erwähnten Kombination im Vergleich zum Referenz-Konzentrationsniveau identifiziert wird, welches in gesunden Kontrollindividuen gemessen wird, dies darauf schließen lässt, dass das Individuum unter kolorektalem Krebs und/oder einem präkanzerogenen Stadium desselben leidet.

3. *In-vitro*-Verfahren nach einem der vorhergehenden Ansprüche, wobei das präkanzerogene Stadium von kolorektalem Krebs fortgeschrittenes kolorektales Adenom ist.

4. *In-vitro*-Verwendung der Kombination [Flt3L und CYFRA21-1] in einer vom Individuum erhaltenen biologischen Probe ausgewählt aus Blut, Serum oder Plasma zur Diagnose von kolorektalem Krebs und/oder einem präkanzerogenen Stadium desselben.

5. *In-vitro*-Verwendung der Kombination [Flt3L und CYFRA21-1 und AREG] oder [Flt3L und CYFRA21-1 und AREG und ErbB4] oder [Flt3L und CYFRA21-1 und AREG und CLEC2C] in einer vom Individuum erhaltenen biologischen Probe ausgewählt aus Blut, Serum oder Plasma nach Anspruch 4, zur Diagnose von kolorektalem Krebs und/oder einem präkanzerogenen Stadium desselben.

6. *In-vitro*-Verwendung eines Kits umfassend zur Bestimmung des Konzentrationsniveaus von mindestens einer Signatur angepasste Reagenzien, ausgewählt aus der Liste: [Flt3L und CYFRA21-1] oder [Flt3L und CYFRA21-1 und AREG] oder [Flt3L und CYFRA21-1 und AREG und ErbB4] oder [Flt3L und CYFRA21-1 und AREG und CLEC2C] in einer vom Individuum erhaltenen biologischen Probe ausgewählt aus Blut, Serum oder Plasma zur Diagnose von kolorektalem Krebs und/oder einem präkanzerogenen Stadium desselben.

7. *In-vitro*-Verwendung nach Anspruch 6, wobei das präkanzerogene Stadium von kolorektalem Krebs fortgeschrittenes kolorektales Adenom ist.

## Revendications

1. Procédé *in vitro* pour le diagnostic du cancer colorectal et/ou de son stade précancéreux qui comprend : a) la mesure du niveau de concentration de la combinaison [Flt3L et CYFRA21-1] dans un échantillon biologique obtenu du sujet, sélectionné à partir de sang, de sérum ou de plasma ; et b) dans lequel, si une déviation ou une variation du niveau de concentration d'au moins la combinaison [Flt3L et CYFRA21-1] est identifiée, en comparaison avec le niveau de concentration de référence mesuré chez des sujets témoins sains, cela est révélateur que le sujet souffre de cancer colorectal et/ou d'un stade précancéreux.

2. Procédé *in vitro* pour le diagnostic du cancer colorectal et/ou de son stade précancéreux, selon la revendication 1, qui comprend : a) la mesure du niveau de concentration d'au moins la combinaison [Flt3L et CYFRA21-1 et AREG], ou de la combinaison [Flt3L et CYFRA21-1 et AREG et ErbB4] ou de la combinaison [Flt3L et CYFRA21-1 et AREG et CLEC2C] dans un échantillon biologique obtenu du sujet sélectionné à partir de sang, de sérum ou de plasma ; et b) dans lequel, si une déviation ou une variation du niveau de concentration d'au moins une combinaison citée dans l'étape a) est identifiée, en comparaison avec le niveau de concentration de référence mesuré chez des sujets témoins sains, cela est révélateur que le sujet souffre de cancer colorectal et/ou d'un stade précancéreux.

3. Procédé *in vitro,* selon l'une quelconque des revendications précédentes, dans lequel le stade précancéreux du cancer colorectal est un adénome colorectal avancé.

4. Utilisation *in vitro* de la combinaison [Flt3L et CYFRA21-1] dans un échantillon biologique obtenu du sujet sélectionné à partir de sang, de sérum ou de plasma pour le diagnostic du cancer colorectal et/ou de son stade précancéreux.

5. Utilisation *in vitro* de la combinaison [Flt3L et CYFRA21-1 et AREG], ou [Flt3L et CYFRA21-1 et AREG et ErbB4], ou [Flt3L et CYFRA21-1 et AREG et CLEC2C] dans un échantillon biologique obtenu du sujet sélectionné à partir de sang, de sérum ou de plasma, selon la revendication 4, pour le diagnostic du cancer colorectal et/ou de son stade précancéreux.

6. Utilisation *in vitro* d'un kit comprenant des réactifs adaptés pour la détermination du niveau de concentration d'au moins une signature sélectionnée de la liste : [Flt3L et CYFRA21-1], ou [Flt3L et CYFRA21-1 et AREG], ou [Flt3L et CYFRA21-1 et AREG et ErbB4], ou [Flt3L et CYFRA21-1 et AREG et CLEC2C] dans un échantillon biologique obtenu du sujet sélectionné à partir de sang, de sérum ou de plasma pour le diagnostic du cancer colorectal et/ou de son stade précancéreux.

7. Utilisation *in vitro,* selon la revendication 6, dans laquelle le stade précancéreux du cancer colorectal est un adénome colorectal avancé.
